# EUROPEAN PATENT APPLICATION

(11) **EP 4 603 035 A1**
(43) Date of publication of application: **20.08.2025**
(21) Application number: 25158100.5
(22) Date of filing: 14.02.2025
(51) Int. Cl.: A61B 17/16, A61B 17/04, A61B 17/17, A61B 17/86, A61B 17/88

(54) **PIN GUIDE AND DRILL GUIDE SYSTEMS**

(30) Priority: 16.02.2024 US 202463554399 P
(71) Applicant: International Life Sciences, LLC d/b/a Artelon, Marietta, Georgia 30067 (US)
(72) Inventor: SLUDER, Justin C., Marietta Georgia, 30067 (US); CAIN, David Brett, Marietta Georgia, 30067 (US); GOEDE, Todd E., Marietta Georgia, 30067 (US); SMITH, Aaron C., Marietta Georgia, 30067 (US)
(74) Representative: V.O.

(57) **Abstract**

Orthopedic systems using a bone peg. In one example an orthopedic system includes a guide pin, a bone peg, a cutter, and a cutting guide. The bone peg may be used for guiding the guide pin through the cutting guide and into bone, with the guide pin used to guide the cutter for forming a hole in the bone. The bone peg may also be inserted into the hole after the hole is formed and used for additional beneficial purposes.

## Description

### RELATED FIELDS

Orthopedic systems, including systems using a bone peg positioned in a hole formed in a bone.

### BACKGROUND

Guide pins, cutters, and cutting guides are all known components of systems for forming holes in bones in the orthopedic arts. There remains, however, room for improvement.

### SUMMARY

In one example, an orthopedic system includes a guide pin, a bone peg, a cutter, and a cutting guide. The bone peg comprises a head, a shaft, and a cannula, the cannula configured to receive and guide the guide pin. The cutter comprises a cannula configured to receive and be guided by the guide pin. The cutting guide comprises a cannula configured to alternatively receive and guide: (i) the bone peg; and (ii) the cutter. The orthopedic system can be used in an orthopedic method including inserting the guide pin into a bone, with insertion of the guide pin being guided by and passing through a cannulation in the bone peg, with the bone peg in turn being inserted in the cannula of a cutting guide positioned on the bone. Next, after insertion of the guide pin into the bone, the bone peg may be removed from the cutting guide. Next, after removal of the bone peg, a cannulated cutter may be positioned over the guide pin and used to form a hole in the bone, with the guide pin guiding the formation of the hole in the bone by the cannulated cutter. Next, after forming the hole in the bone, the cannulated cutter may be removed from the hole. Next, after removing the cannulated cutter from the hole, the shaft of the bone peg may be inserted into the hole in the bone such that the head of the bone peg extends upwardly away from the hole in the bone.

After insertion of the bone peg into the hole in the bone, the bone peg may be used for a variety of beneficial purposes. For instance, the head of the bone peg may provide a visual indication of a trajectory of the hole in the bone. As another example, at least a portion of the bone peg may be of a radio-opaque material, and the bone peg may be used to provide a visual indication of the bone hole (such as its position, trajectory, and/or depth) when the bone is imaged using a surgical imaging system. As another example, the head of the bone peg may be used to resist soft tissue from moving over the bone hole. As additional examples, suture may be secured to and/or tensioned using the head of the bone peg.

### BRIEF DESCRIPTION OF FIGURES

FIG. 1 shows an example of an orthopedic system.
FIG. 2 shows a guide pin from the orthopedic system of FIG. 1.
FIG. 3 shows a bone peg from the orthopedic system of FIG. 1.
FIG. 4 shows a cutter from the orthopedic system of FIG. 1.
FIG. 5 shows a cutting guide from the orthopedic system of FIG. 1.
FIGS. 6-13 show an example of an orthopedic method.

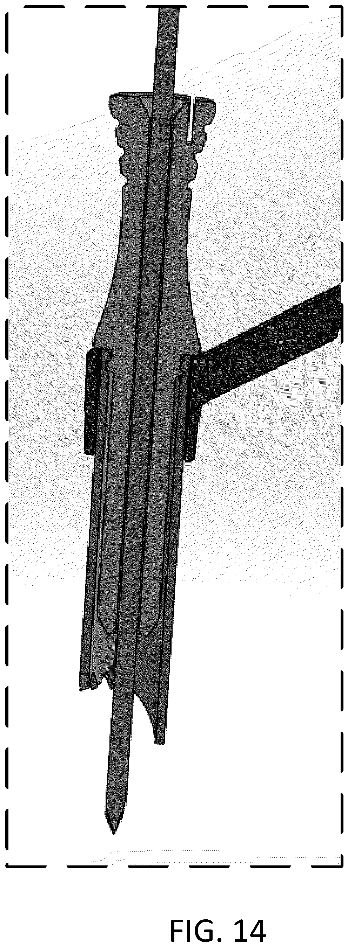

FIG. 14 shows the guide pin, bone peg, and cutting guide from the system of FIG. 1 in cross-section, with the guide pin inserted in the bone peg and the bone peg inserted in the cutting guide.

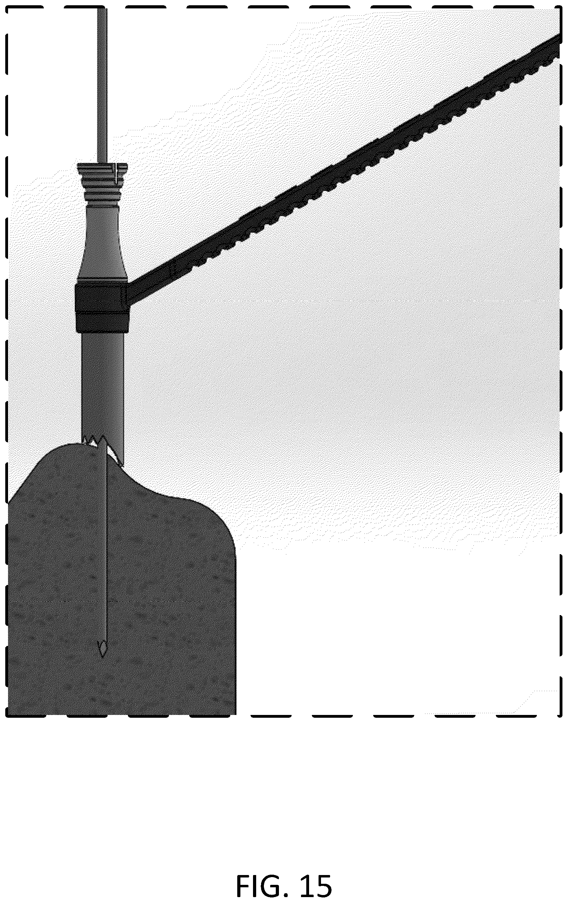

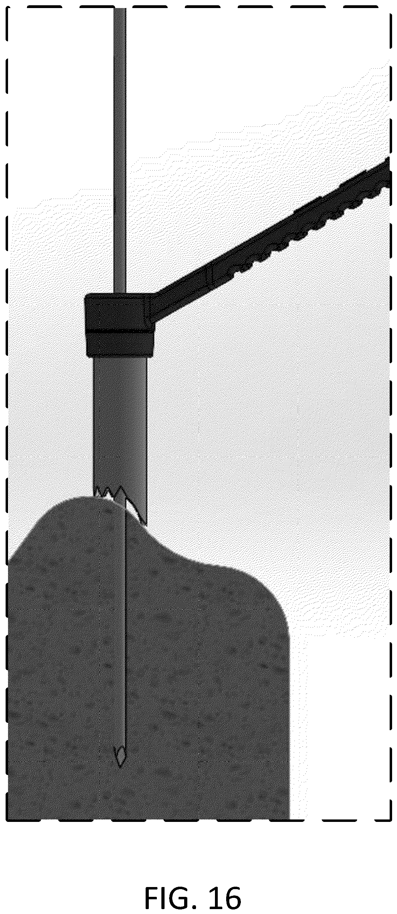

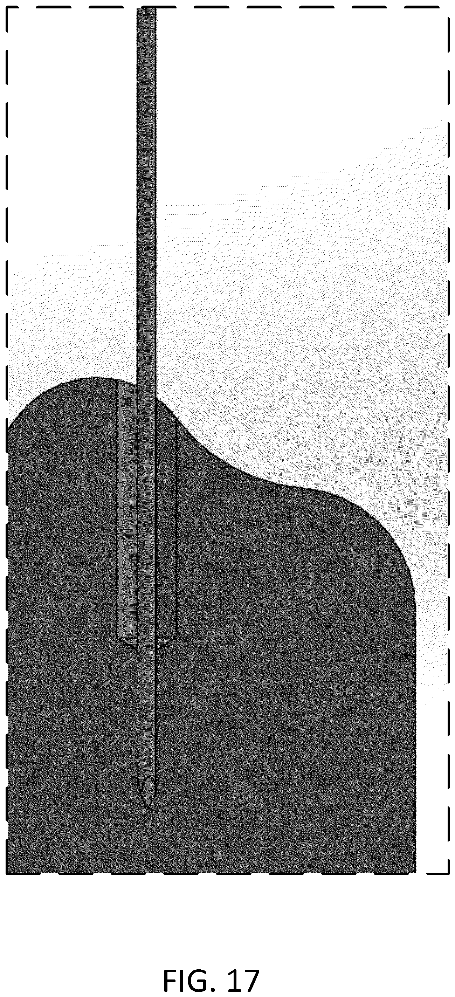

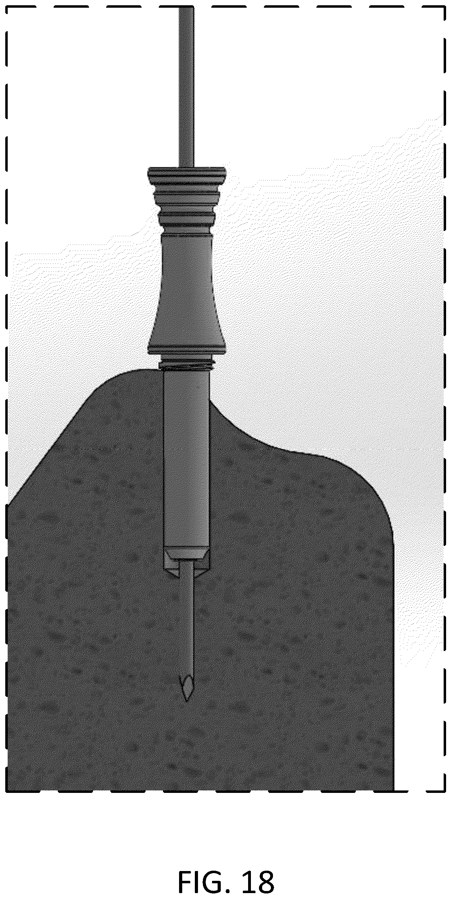

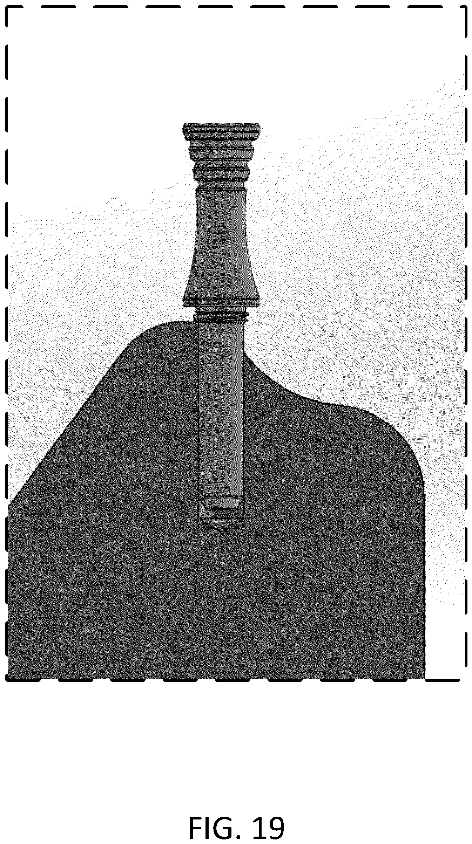

FIGS. 15-19 are additional images of the orthopedic method of FIGS. 6-13.
FIG. 20 shows an example of the use of three bone pegs to provide visual indications of bone hole trajectory and to resist soft tissue from moving over the bone holes.
FIG. 21 shows an example of the use of a bone peg to temporarily secure a suture.

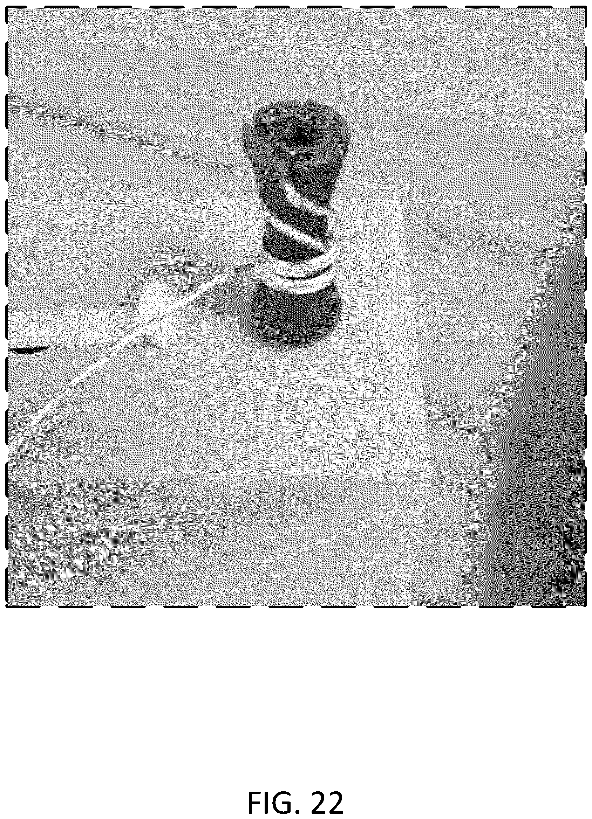

FIG. 22 shows an example of the use of a bone peg to tension a suture.

### DETAILED DESCRIPTION

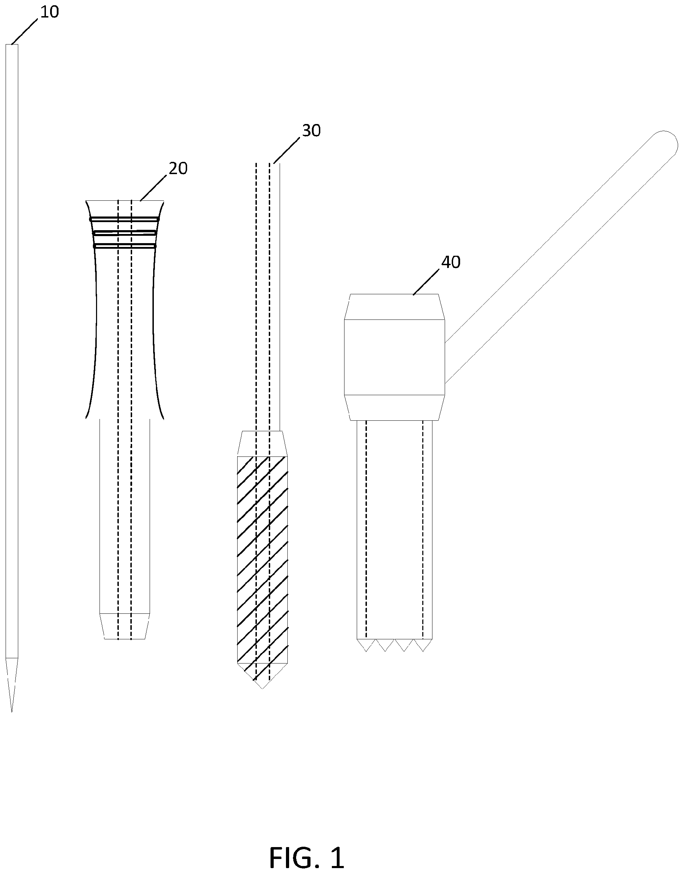

FIG. 1 shows an example of an orthopedic system including a guide pin 10, a bone peg 20, a cutter 30, and a cutting guide 40. The bone peg 20, cutter 30, and cutting guide 40 are all cannulated, with the cannulas of the bone peg 20 and cutter 30 both configured (in size and shape) to receive the guide pin 10, and the cannula of the cutting guide 40 configured (in size and shape) to alternatively receive either the bone peg 20 or the cutter 30.

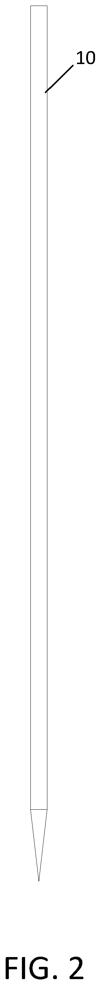

FIG. 2 shows the guide pin 10 of the orthopedic system of FIG. 1.

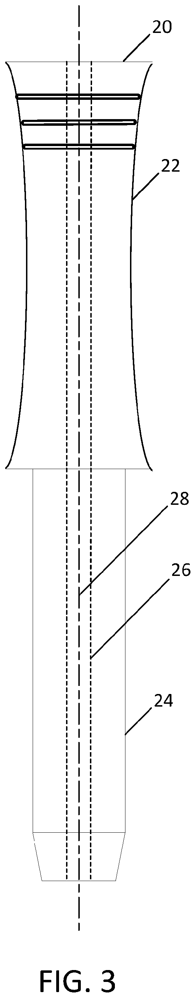

FIG. 3 shows the bone peg 20 of the orthopedic system of FIG. 1. The bone peg 20 in this example includes a head 22, a shaft 24, and a cannula 26 extending through the head 22 and shaft 24. The cannula 26 is configured to receive and guide the guide pin 10. The head 22, shaft 24, and cannula 26 are all aligned along a longitudinal axis 28 of the bone peg 20. The shaft 24 is configured (in size and shape) to be inserted into a cannula of the cutting guide 40, and is also configured (in size and shape) to be inserted into a bone hole formed by cutter 30. An outer diameter of the guide pin 10 can be approximately the same as an inner diameter of a cannula 26 of the bone peg 20. The outer diameter of the guide pin 10 can be approximately the same as an inner diameter of a cannula of the cutter 30. An inner diameter of a canula of the cutting guide 40 can be approximately the same as an outer diameter of a shaft of the bone peg 20. The inner diameter of the canula of the cutting guide 40 can be approximately the same as an outer diameter of a cutting portion of the cutter 30. In this context, "approximately the same" means 10% or less difference. When the shaft 24 is inserted into a bone hole, the head 22 of the bone peg 20 will extend upwardly and away from the hole in the bone. In the examples shown in the figures, the head 22 is a concave cylinder, which may make it easier to handle in the operating environment and facilitate some of the other functions discussed below (such as acting as a soft tissue dam or securing / tensioning suture).

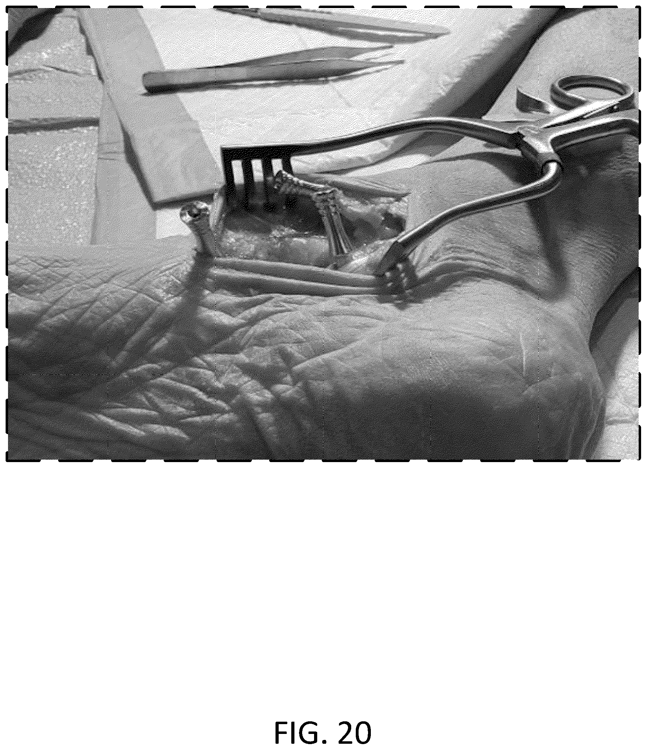

In some implementations, when the shaft 24 of the bone peg 20 is inserted into a hole in a bone, the head 22 of the bone peg may provide a visual indication of a trajectory of the hole in the bone. For example, FIG. 20 shows three bone pegs inserted into three bone holes, providing a clear visualization to the surgeon and other operating room personnel of the trajectories of the three bone holes, which may otherwise not be easy to visually determine from just viewing the holes themselves.

In some implementations, when the bone peg 20 is inserted into a hole in a bone, the head 22 of the bone peg 20 may function as a soft tissue dam surface that resists soft tissue from moving over the hole in the bone. For example, FIG. 20 shows three bone pegs inserted into bone holes, with the heads of the bone pegs helping to prevent soft tissue form moving over and covering the bone holes.

In some implementations, at least a portion of the bone peg 20 may be formed of a radio-opaque material. For instance, the shaft 24 may be formed of a radio-opaque material. In these instances, when the bone peg 20 is inserted in a hole in a bone, the bone peg 20 can provide a visual indication of the hole in the bone (such as the holes position, trajectory, and depth) when the bone is imaged using a surgical imaging system such as a CT scanner or other x-ray device.

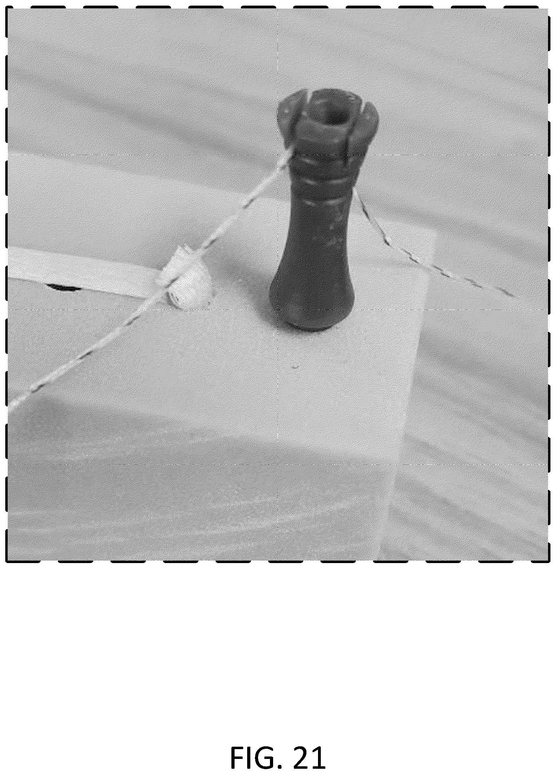

In some implementations, the head 22 of the bone peg may be used to temporarily secure suture during an operation. The head 22 of the bone peg may include one or more suture retention sites such as the suture cleats shown in FIG. 21 for securing suture or may include other features for accomplishing the same or similar functions, such as o-rings or push button based mechanisms that temporarily retain suture on the head 22. In some implementations, the head 22 of the bone peg 20 may be used to tension suture. For instance, the suture may be wrapped around the head 22 of the bone peg 20 and the bone peg 20 may be rotated in the bone hole 52 such that the suture winds around the head 22 of the bone peg 20, thereby tensioning the suture.

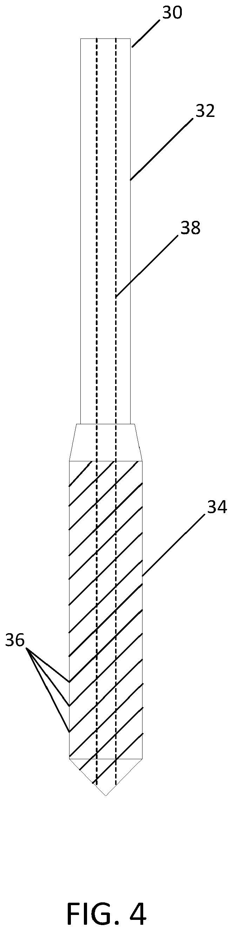

FIG. 4 shows the cutter 30 of the orthopedic system of FIG. 1. In this example the cutter 30 is a drill bit including a shank 32 and a cutting portion 34, with cutting flutes 36 spiraling around the cutting portion 34. The outer diameter of the cutting portion 34 is approximately the same as the outer diameter of the shaft 24 of the bone peg 20. In this context, "approximately the same" means 10% or less difference. The cutter 30 in this example has a cannula 38 that is configured to receive and be guided by the guide pin 10.

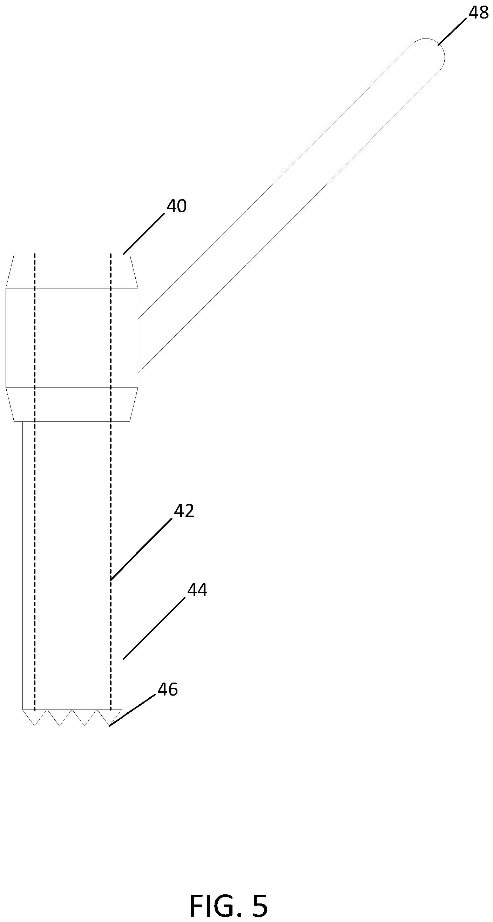

FIG. 5 shows the cutting guide 40 of the orthopedic system of FIG. 1. The cutting guide 40 in this example includes a cannula 42 configured to alternatively receive and guide the bone peg 20 and the cutter 30. The cannula 42 of the cutting guide 40 extends through a body 44 including a toothed end 46 that facilitates maintaining the position of the cutting guide 40 on a bone surface. The cutting guide 40 in this example also includes a handle 48.

Although not shown in the figures, the bone peg 20 may be secured in the cannula 42 of the cutting guide 40 in a variety of fashions. Non-limiting examples include: superficial threads on the bone peg 20 and/or the cutting guide 40; an o-ring on the bone peg 20; a locking pin and mating feature; a clip on the cutting guide 40; a ball detent; and/or a push button.

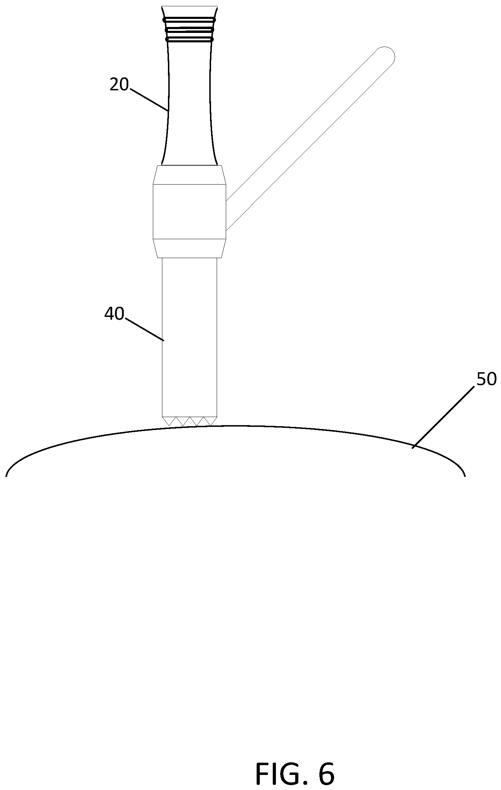

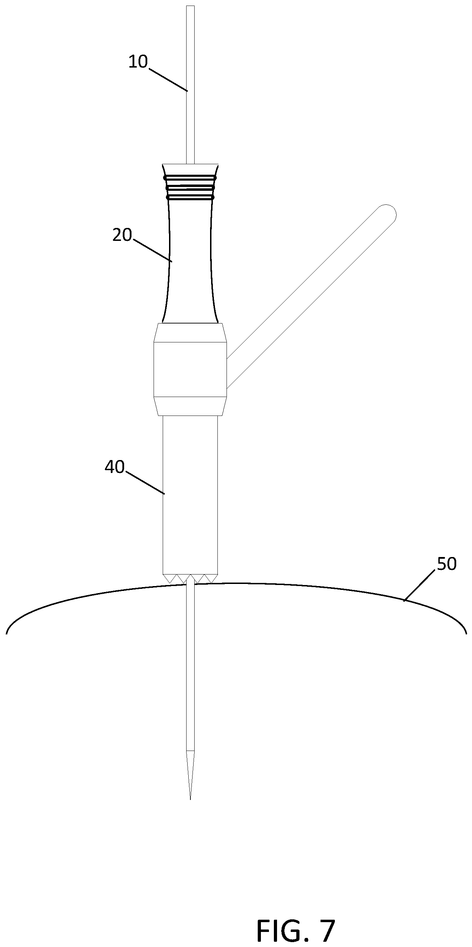

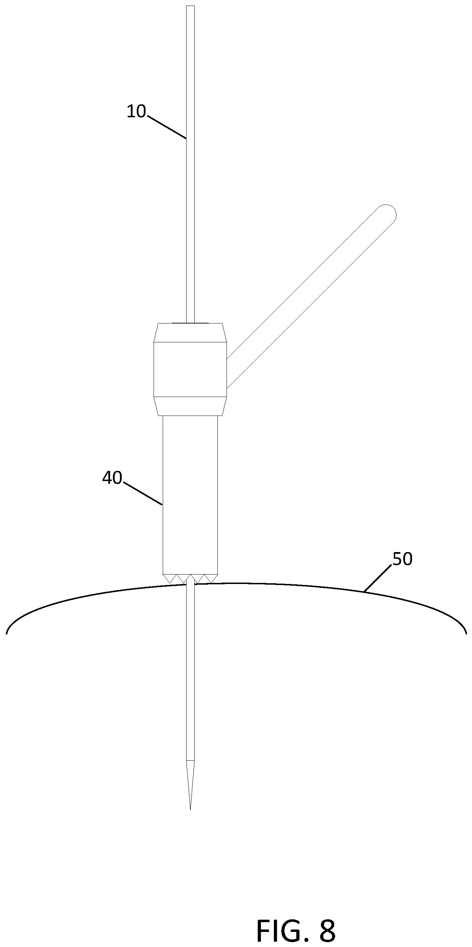

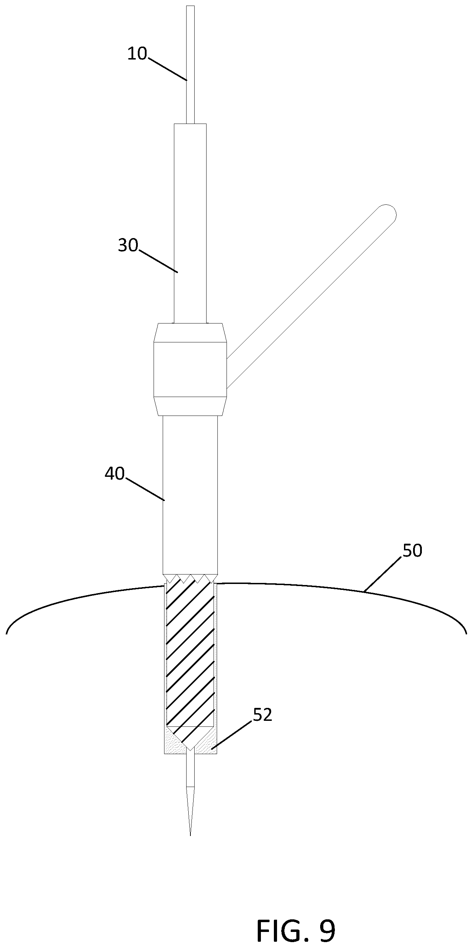

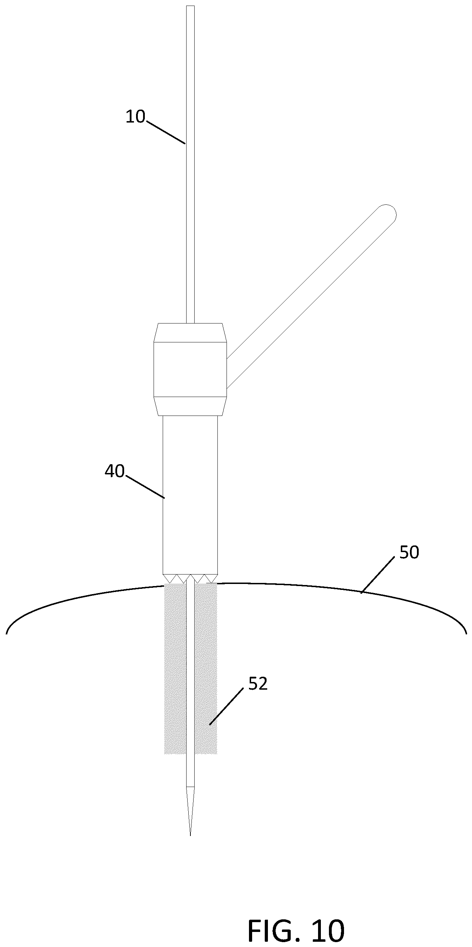

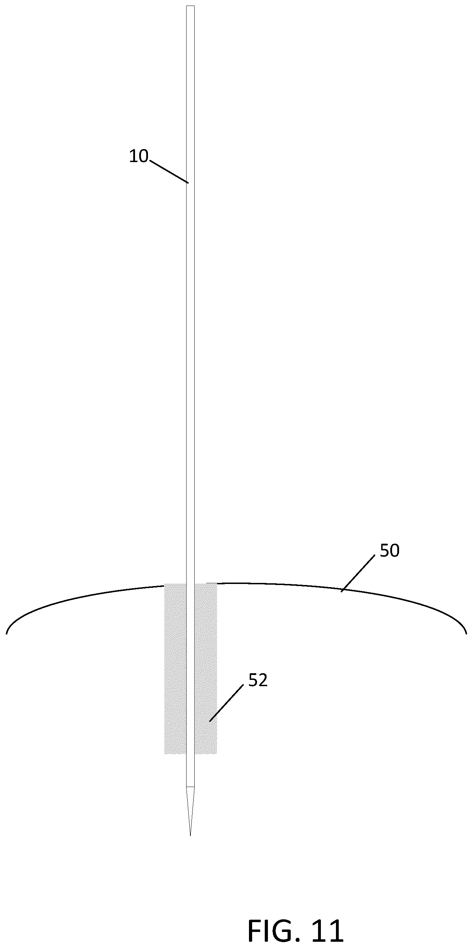

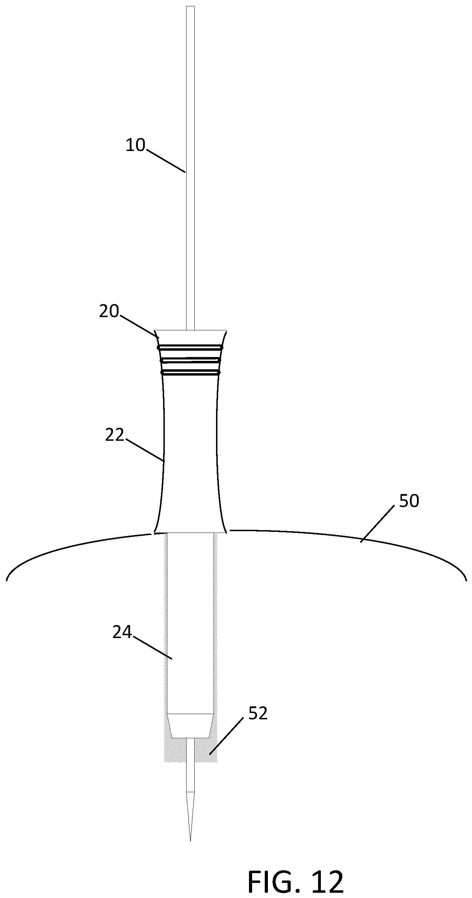

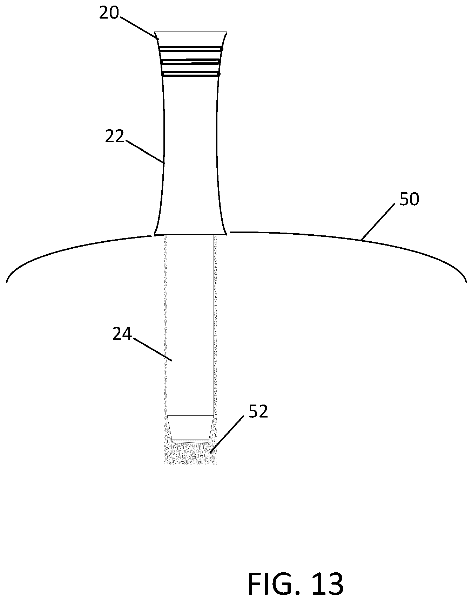

FIGS. 6-13 show an example of an orthopedic method using the orthopedic system of FIGS. 1-5. In FIG. 6, the cutting guide 40 is shown positioned on a bone 50, with the bone peg 20 inserted in the cannula of cutting guide 40. In FIG. 7, the guide pin 10 is inserted into the bone 50. Insertion of the guide pin 10 is guided by and passes through the cannulation in bone peg 20. FIG. 8 shows the bone peg 20 removed from the cutting guide 40 after insertion of the guide pin 10 into the bone 50. Next, as shown in FIG. 9, the cannulated cutter 30 may be positioned over the guide pin 10 and be used to form a hole 52 in the bone 50. Both the guide pin 10 and the cannula 42 of the cutting guide 40 guide the formation of the hole 52 in the bone 50 by guiding the cannulated cutter 30. Next, as shown in FIG. 10, the cannulated cutter 30 may be removed from the bone hole 52 and from the cutting guide 40. Note that although the figures show the guide pin 10 remaining in the bone hole 52 after removal of the cutter 30, in other implementations the guide pin 10 may be removed along with the cutter 30. Next, as shown in FIG. 11, the cutting guide 40 may be removed from the bone 50, leaving just the guide pin 10 in the bone hole 52. Next, as shown in FIG. 12, the shaft 24 of the bone peg 20 may be inserted into the bone hole 52 such that the head 22 of the bone peg 20 extends upwardly away from the bone hole 52. As shown in FIG. 12, the outer diameter of the shaft 24 of the bone peg 20 is approximately the same as the inner diameter of the hole 52 in the bone 50. In this context, "approximately the same" means 10% or less difference. As shown in FIG. 13, the bone peg 20 may remain in the bone hole 52 after the guide pin 10 is removed from the bone 50.

As shown in FIG. 13 and in FIG. 20, when the bone peg 20 is inserted in the hole 52 in the bone 50, the head 22 of the bone peg 20 provides a visual indication of a trajectory of the hole 52 in the bone 50. For instance, since the head 22 of the bone peg 20 extends along the same longitudinal axis as the shaft 24 of the bone peg 20, the head 22 can provide a visual indication of the trajectory of the shaft 24 of the bone peg 20 (which will be the same as the trajectory of the bone hole 52 since he outer diameter of the shaft 24 is approximately the same as the inner diameter of the hole 52).

Although not shown in the figures, the bone peg 20 may be subsequently removed from the bone hole 52 and a fastener may be installed into the bone hole 52 (along with a suture, a surgical mesh, or other construct for fixation to the bone).

## Claims

1. An orthopedic system comprising:
(a) a guide pin;
(b) a bone peg comprising a head, a shaft, and a cannula, the cannula configured to receive and guide the guide pin;
(c) a cutter comprising a cannula configured to receive and be guided by the guide pin; and
(d) a cutting guide comprising a cannula configured to alternatively receive and guide: (i) the bone peg; and (ii) the cutter.

2. The orthopedic system of claim 1, wherein the cannula of the cutting guide is sized and/or shaped to alternatively receive and guide either the bone peg or the cutter.

3. The orthopedic system of claim 1 or 2, wherein the cannulas of the bone peg and the cutter are both configured, in particular in size and shape, to receive the guide pin.

4. The orthopedic system of claim 1, 2 or 3, wherein the shaft is configured, in particular in size and shape, to be inserted into the cannula of the cutting guide and/or a hole formed by the cutter.

5. The orthopedic system of any of claims 1-4, wherein the cutter is configured, in particular in size and shape, to be inserted into the cannula of the cutting guide.

6. The orthopedic system of any of claims 1-5, wherein an outer diameter of the shaft of the bone peg is approximately the same as an outer diameter of a cutting portion of the cutter.

7. The orthopedic system of any of claims 1-6, wherein an outer diameter of the guide pin is approximately the same as an inner diameter of the cannula of the bone peg.

8. The orthopedic system of any of claims 1-7, wherein the outer diameter of the guide pin is approximately the same as an inner diameter of the cannula of the cutter.

9. The orthopedic system of any of claims 1-8, wherein an inner diameter of the canula of the cutting guide is approximately the same as an outer diameter of the shaft of bone peg.

10. The orthopedic system of any of claims 1-9, wherein the inner diameter of the canula of the cutting guide is approximately the same as an outer diameter of a cutting portion of the cutter.

11. The orthopedic system of any of claims 1-10, wherein the bone peg comprises a longitudinal axis, wherein the head, the shaft, and the cannula of the bone peg are all aligned along the longitudinal axis.

12. The orthopedic system of any of claims 1-11, wherein at least a portion of the bone peg comprises a radio-opaque material.

13. The orthopedic system of any of claims 1-12, wherein the head comprises a soft tissue dam surface.

14. The orthopedic system of any of claims 1-13 wherein the head comprises at least one suture retention site.

15. The orthopedic system of any of claims 1-14, wherein the bone peg is configured to be secured in the cannula the cutting guide, in particular by superficial threads on the bone peg and/or the cutting guide, an o-ring on the bone peg, a locking pin and mating feature, a clip on the cutting guide, a ball detent, and/or a push button.
